# EUROPEAN PATENT SPECIFICATION

(11) **EP 3 748 361 B1**
(45) Date of publication and mention of the grant of the patent: **06.10.2021**
(21) Application number: 18714299.7
(22) Date of filing: 29.01.2018
(51) Int. Cl.: G01N 33/574

(54) **METHOD AND SYSTEM FOR SELECTING, MANIPULATING AND ISOLATING CIRCULATING TUMOUR CELLS IN BODILY FLUIDS VIA LASER-ASSISTED TRANSFER**
VERFAHREN UND SYSTEM ZUR AUSWAHL, MANIPULATION UND ISOLIERUNG VON ZIRKULIERENDEN TUMORZELLEN IN KÖRPERFLÜSSIGKEITEN DURCH LASERUNTERSTÜTZTE ÜBERTRAGUNG
PROCÉDÉ ET SYSTÈME POUR SÉLECTIONNER, MANIPULER ET ISOLER DES CELLULES TUMORALES CIRCULANTES DANS DES LIQUIDES CORPORELS PAR TRANSFERT ASSISTÉ PAR LASER

(43) Date of publication of application: 09.12.2020
(73) Proprietor: Universidad Politécnica De Madrid, 28040 Madrid (ES)
(72) Inventor: MOLPECERES ÁLVAREZ, Carlos Luis, 28040 Madrid (ES); LAUZURICA SANTIAGO, Sara, 28040 Madrid (ES); MÁRQUEZ FERNÁNDEZ, Andrés, 28040 Madrid (ES); MUÑOZ MARTÍN, David, 28040 Madrid (ES); MORALES FURIÓ, Miguel, 28040 Madrid (ES); COLINA BRITO, Mónica, 28040 Madrid (ES); MARTÍN JIMÉNEZ, Miguel, 28007 Madrid (ES); RAMOS MEDINA, Rocío, 28007 Madrid (ES); MUÑOZ MARTÍN, Andrés, 28007 Madrid (ES); GAYARRE NAVARRO, Javier, 28007 Madrid (ES); MASSARRAH SÁNCHEZ, Tatiana, 28007 Madrid (ES); DEL MONTE MILLÁN, María, 28007 Madrid (ES)
(74) Representative: Elzaburu S.L.P.
(86) International application number: PCT/ES2018/070064
(87) International publication number: WO 2019/145581

(56) References cited:
- US-A1- 2018 112 277
- NATHAN R SCHIELE ET AL: "Laser-based direct-write techniques for cell printing", BIOFABRICATION, vol. 2, no. 3, 12 July 2010 (2010-07-12), page 032001, XP055489710, UK ISSN: 1758-5082, DOI: 10.1088/1758-5082/2/3/032001
- YU DENG ET AL: "Single cell isolation process with laser induced forward transfer", JOURNAL OF BIOLOGICAL ENGINEERING, vol. 11, no. 1, 13 January 2017 (2017-01-13), XP055489711, DOI: 10.1186/s13036-016-0045-0

## Description

### TECHNICAL FIELD

The present invention relates to methods and systems for detecting, manipulating and isolating circulating tumor cells found in organic fluids by laser-assisted transfer.

### BACKGROUND OF THE INVENTION

Biopsies of tumor tissues commonly involve accessibility issues and the use of invasive procedures that result, in most of the cases, in a high degree of discomfort for patients and a considerable rise of expenses for health systems. It is widely accepted that metastatic dynamics are associated to the circulation of subgroups of tumor cells that leave the primary tumor and travel through the peripheral blood colonizing new distant tissues. The metastatic dissemination starts when a subpopulation of tumor cells coming from the primary tumor acquires a mesenchymal-like migratory phenotype during the tumor progression. These cells lose their epithelial properties through a morphogenetic process known as Epithelial-to-Mesenchymal transition, EMT, which results in changes in cell motility and plasticity and allows cells to become invasive and enter the peripheral bloodstream.

The cancer cells traveling through the peripheral blood are known as Circulating Tumor Cells, CTCs. A subgroup of CTCs that is found in the bone marrow and nodes, is known as Disseminated Tumor Cells, DTCs. The molecular analysis and sequencing of these tumor cells provides a powerful tool for disease prognosis and progression monitoring and therefore, the risk of systemic metastasis and relapse would be potentially reduced.

The fact that CTCs can be extracted from peripheral blood allows counting on other sources of biopsies not involving the access to tumor tissues, which provides an accessible and non-invasive technique for genomic analysis. Due to the natural liquid environment of CTCs, the term "liquid biopsy" is usually employed when analyzing these type of cells. Therefore, this type of biopsy involves an advantageous lower invasiveness degree when comparing with conventional biopsies from tumor tissue. In addition, the liquid biopsy can be used for early diagnosis which may lead to a selection of a more suitable treatment. Nevertheless, the concentration of CTCs in cancer early stages is very low (1 CTC per 10⁶ - 10⁹ cells) which implies challenging requirements in terms of sensitivity and specificity of detection methods.

On the other hand, understanding the cancer cellular dynamics at a single cell level is a key step to develop personalized and more effective therapies. The molecular analysis at a single cell level is known as Single Cell Sequencing, SCS, and allows to obtain a complete genomic, transcriptomic and epigenomic characterization of the amplified genetic material. This advanced technology requires the precise isolation of the single cell of interest. Thus, the combination of the concept of liquid biopsy from CTCs and DTCs with the molecular analysis at a single cell level, SCS, currently represents an inflexion point for oncologic research. In addition, it is known that aggregations of tumor cells, known as CTC clusters (more than two or three cells), that travel together in the bloodstream have a greater predisposition of forming distal metastasis than single CTCs. Therefore, the molecular analysis of precisely isolated CTC clusters is also interesting to develop personalized treatments capable to stop the metastatic progression. The possibility to choose between the isolation of single cells or clusters and even more, the possibility to manipulate cluster in such a manner that the disaggregation of a pre-selected cluster in different single cells is performed would give to oncology researchers a powerful tool allowing to undertake parallel analysis at a single cell and a cluster level.

Due to the low concentration of CTCs, the initial liquid specimen containing CTCs must be enriched. The subsequent CTCs isolation from the enriched liquid specimen remains a technically challenging issue. Immunomagnetic Separation, IMS, is one of the most common method to isolate CTCs. This method uses antibodies coating paramagnetic beads which bind antigens present in the cells. For positive selection, which comprises the direct capturing of the CTCs among the rest of cells in the liquid specimen, the expression of the epithelial cell adhesion molecule, EpCAM, antigen is used as biological marker. This antigen is a surface protein expressed on epithelial cells. On the contrary, for negative selection, the expression of CD45 antigen is employed to differentiate hematopoietic cells from epithelial cells. Due to the specificity of CD45 for leukocyte populations this antigen allows the recuperation of only the CTCs population in the final liquid specimen. The CD45 antigen is expressed on hematopoietic cells, thus it can differentiate hematopoietic from epithelial cells. Besides that, there is an unmet need to increase the purity and the concentration of the CTCs fraction as well as to maintain the cellular viability in order to facilitate not only the molecular characterization but also the possibility of performing in-vitro studies. Despite the fact that the negative selection does not compromise in any case the CTC integrity since they are not biologically marked, additional separation steps may reduce their concentration, purity and compromise their cellular viability depending on the employed separation method. The US Food and Drug Administration, FDA approved CellSearch® manufactured by Veridex is based on the inmunomagnetic separation using EpCAM expression. Other inmunocytogical based methods using epithelial features of CTCs are EPhithelial inmmunoSpot (EPISPOT), a microdevice CTC chip called Ephesia and the inmmunomagnetic separation technology called MagSweeper. Reviews of the current technologies for detecting CTCs can be found in: "Circulating tumor cells: approaches to isolation and characterization", M. Yu et al., J. Cell Bio. 192: 3, 373-382 (2011); and "Single-Cell Sequencing Technology in Oncology: Applications for Clinical Therapies and Research", B. Ye et al., Anal Cell Pathol, 2016, DOI: 10.1155/2016/9369240.

On the other hand, it is also known that EpCAM is not suitable as biological marker to detect CTCs during Epithelial-to-Mesenchymal Transition, EMT. In that sense, increasing the possibility of isolating CTCs in any of its phases, even undergoing EMT is a relevant current challenge. Consequently, a methodology allowing the isolation of all CTCs subpopulations regardless of their epithelial or mesenchymal features is needed.

A good alternative to enable the isolation of a single CTC or CTC clusters without discrimination in terms of its epithelial or mesenchymal features is the laser-capture-microdissection, LCM, technology. This technology has been traditionally used to isolate cells coming from fresh tissues. Nevertheless, some disadvantages concerning possible UV cellular damages in RNA/DNA caused by the laser irradiation are repeatedly reported. In addition, this technology also has complex requirements related to sample preparation. Generally, the cellular media must be solidified for applying LCM so the natural cellular environment is necessarily modified.

A second alternative involving the use of a laser technology that overcomes the limitations of UV damage and cellular environment transformation is the laser-induced forward transfer, LIFT, technique applied to liquid specimens. In this technology, a transparent substrate is used to support the liquid specimen. In order to avoid direct illumination damage, it is known that a radiation absorbent intermediate layer is located in the middle of the laser beam path between the transparent support and the biological liquid specimen. This intermediate layer coats the transparent support and has the role of absorbing the largest part of the laser radiation. As a consequence of the energy absorption, a portion of the liquid containing the biological sample is thermally or mechanically propelled. This intermediate layer has been always understood as a coating regardless of the type of material employed, which can be metals or polymers. Consequently, a previous deposition step of this radiation absorbent intermediate layer onto the transparent support is needed. Thus, when using polymeric semitransparent materials for the intermediate layer, spin coating is the preferred methodology for its deposition. An additional step of drying is also compulsory for the complete preparation of the substrate holding the biological liquid specimen. Nathan R Schiele (Biofabrication, vol. 2, no. 3, 12 July 2010, page 032001) discloses the LIFT technique applied to liquid specimens.

A simplification of LIFT when using polymeric absorbent intermediate layers is proposed in this document. This simplification, called simplified laser-assisted transfer in this disclosure, permits to reduce the number of steps required for the substrate preparation when LIFT is used. In addition, a method for detection and manipulation of CTCs and DTCs using the laser-assisted transfer technique is also proposed in this document.

### BRIEF DESCRIPTION OF THE INVENTION

The present invention relates to a method according to independent claim 1 and system according to independent claim 12 for detecting and manipulating tumor cells, more particularly CTCs and DTCs, found in organic fluids. Manipulating may comprise both the selective movement of cells and/or their isolation from the rest of cells contained in the organic fluid. The liquid specimen of the organic fluid contains an enriched population of tumor cells. Other populations of cells grouped in the so called non-tumor cells are also presented in the liquid specimen. A fluorescent staining of at least one of the cell populations (tumor and non-tumor cells) present in the liquid specimen is required to identify by means of optical and fluorescence microscopy the location of the cells to be isolated by a simplified laser-assisted transfer process.

The simplified laser-assisted transfer is achieved by using a donor substrate that comprises a semi-transparent polymeric adhesive tape adhered to a transparent support. The semi-transparent polymeric adhesive tape is partially transparent to visible light and absorbent to the laser wavelength. The liquid specimen is dispensed and spread onto the semi-transparent polymeric adhesive tape. The donor substrate is arranged in such a manner that an impinging laser beam propagates through the transparent support, in first place, and through the semi-transparent polymeric adhesive tape, in second place. This arrangement of the donor substrate respect to the beam path allows the complete absorption of the laser radiation before reaches the liquid specimen. A receiving substrate is placed facing the surface of the donor substrate where the liquid specimen is spread maintaining a separation distance between them.

For manipulation of single cells or cluster of cells, an imaging system identifies the tumor cells or the non-tumor cells selected for manipulating, then the laser beam is focalized on the interface between the transparent support and the semi-transparent polymeric adhesive tape just above such location. The laser beam energy is completely absorbed by the semi-transparent polymeric adhesive tape giving place to the formation of a blister on the polymeric adhesive tape and where the blister mechanically generates a perturbation on the liquid specimen with the consequent movement of the selected cells. Said movement can result in selected cells being displaced within the liquid specimen. In some cases said movement induces the disaggregation of a cluster of cells in such a manner that single cells originally forming the cluster can be subsequently selected for isolation. In a particularly interesting application, movement of the cells results in a portion of the liquid specimen containing the selected tumor cells or non-tumor cells being propelled towards the receiving substrate thus enabling isolation of the cells. The propelled portion of liquid can contain a single cell or a cell cluster. As the most interesting application of this method, the selection and isolation of non-fluorescent stained CTCs is highlighted. This application receives the name of "laser negative selection" in this disclosure and results more attractive due to two reasons: first, the laser processing time is shorter than in the positive selection because of the very low concentration of CTCs in the liquid specimen, and second, the fact that keeping unaltered the CTCs without using fluorescent dyes would preserve the integrity of these cells in a higher level.

### BRIEF DESCRIPTION OF THE DRAWINGS

Figure **1** is a schematic illustration of the transparent support of the donor substrate;
Figure **2** schematically illustrates the semi-transparent polymeric adhesive tape comprising of the semi-transparent thermoplastic polymer sheet and the transparent adhesive polymer layer;
Figure **3** shows a schematic illustration of the semi-transparent polymeric adhesive tape adhered to the transparent support wherein both elements comprise the donor substrate;
Figure **4** schematically illustrates the arrangement of the liquid specimen containing the two cell populations with respect to all the components of the donor substrate;
Figure **5** schematically illustrates a preferred arrangement of the donor substrate, the liquid specimen, the receiving substrate and the two pieces employed to separate the liquid specimen from the receiving substrate;
Figure **6** shows a schematic illustration of the arrangement of the liquid specimen with respect to the objective lens of the optical and fluorescence microscopy system;
Figure **7** schematically illustrates the arrangement of the focalized laser beam with respect to the donor substrate, the liquid specimen and the receiving substrate;
Figure **8** shows a schematic illustration of the liquid specimen with the two populations of cells, wherein one of them is fluorescent stained and the other one is not stained, with respect to the donor and receiving substrate and the objective lens of the microscopy system;
Figure **9** shows an illustration of the so called laser negative selection wherein the laser beam induced a blister that propels a droplet containing a single non-stained tumor cell.
Figure **10** shows an illustration of the so called laser negative selection wherein the laser beam induced a blister that propels a droplet containing a cluster of non-stained tumor cells.

### DETAILED DESCRIPTION OF THE INVENTION

In the present disclosure, the expression "organic fluid" refers to liquids with a high probability to find circulating tumor cells, CTCs, or disseminated tumor cells, DTCs, such as for example, peripheral blood flow and bone marrow. Additionally, the expression "tumor cells" refers to CTCs and DTCs. Likewise, the expression "non-tumor cells" refers to different cell populations not being CTCs and DTCs that are present in the liquid specimen after the enrichment process. In the case that the liquid specimen is obtained from the peripheral blood flow or bone marrow, hematopoietic cells constitute the largest population even after the enrichment and therefore, are considered non-tumor cells in this context.

According to the previous glossary of terms, the liquid specimen contains two differentiated cell populations called tumor and non-tumor cells where the tumor population is necessarily enriched for applying this method. In addition, a fluorescent staining of at least one of the populations is also required to identify the cells to be isolated by means of the simplified laser-assisted transfer process. The staining process is performed using specific fluorescent antibodies to the cell populations and can be carried out according to one of three alternatives: the first one, where the stained population corresponds to the tumor cells; the second one, where the stained population corresponds to non-tumor cells and the third one, where both populations are stained with different florescence dyes. The fluorescent staining of CTCs and DTCs involves the use of the epithelial cell adhesion molecule, EpCAM, antigens. In the case of non-tumor cells, the fluorescent staining involves the use of CD45, the leukocytes common antigen, LCA, which is expressed on hematopoietic cells. In a preferred embodiment of this method, the liquid specimen is enriched using the method of cellular isolation by density gradient centrifugation (Ficoll) and mechanical filters while fluorescent staining is performed with EpCAM-FITC for tumor cells and CD45 PE for non-tumor cells. After the enrichment process or as consequence of it, the liquid specimen containing the processed cells results in a suspension of the cells in a biocompatible liquid medium or cell culture liquid medium. Different options for biocompatible media such as the RPMI-1640 medium supplemented with fetal bovine serum (FBS), L glutamine, Beta Mercaptoethanol and antibiotics (penicillin and streptomycin); 2 % (w/v) sodium alginate and 0.5-1.5 % (w/w) methylcellulose can be used for this method.

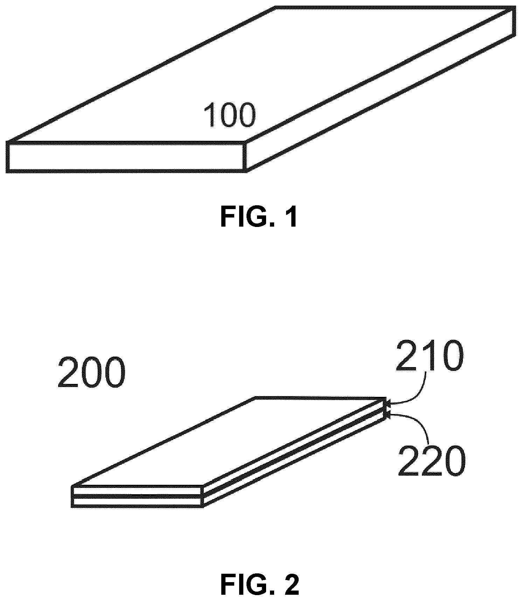

A transparent support is required as a base for the different elements of the donor substrate. The transparency requirement is due to two reasons: first, the necessity of inspection of the liquid specimen by optical and fluorescence microscopy for selecting the location where the laser beam is focused for propelling the liquid specimen and second, for minimizing the absorption of part of the beam radiation by the transparent support which has effects during the blister generation. In a preferred embodiment of this method, the transparent support is a thin flat piece of glass with similar optical and morphological properties in both surfaces of the piece. In a more preferred embodiment of this method, the transparent support is a 1-mm thick soda-lime glass. Figure 1 shows a schematic illustration of the transparent support 100.

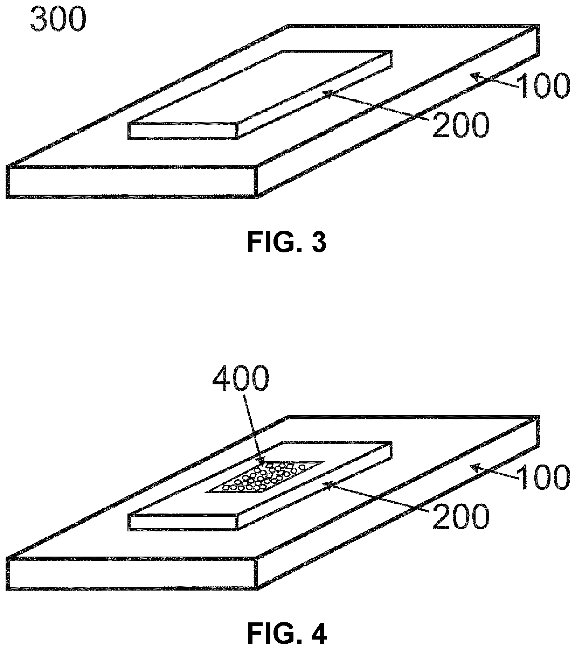

The donor substrate is also comprised by a component that absorbs the laser energy. A semi-transparent polymeric adhesive tape has the role of laser energy-absorbent and at the same time allows the visual inspection of the liquid specimen due to its semi-transparent condition to the visible light. The semi-transparent polymeric adhesive tape has at least a sheet of a semi-transparent thermoplastic polymer and a transparent layer of an adhesive polymer. In a preferred embodiment of this method, the semi-transparent thermoplastic material is a semi-transparent thermoplastic polyimide with a range of thickness between 10 and 100 µm and the transparent adhesive polymer corresponds to a transparent adhesive silicone of a thickness ranging from 10 to 100 µm. Figure **2** shows a schematic illustration of the structure of the polymeric adhesive tape **200** wherein the sheet of the semi-transparent thermoplastic polymer **210** and the layer of the transparent adhesive polymer **220** are represented. In order to prepare the donor substrate **300,** the semi-transparent polymeric adhesive tape **200** is adhered to the transparent support **100** as shown in Figure **3**, with the adhesive surface of the tape **220** in contact with the transparent support **100**.

A volume in the range of tens of microliters of the liquid specimen **400** is dispensed and spread onto the semi-transparent thermoplastic polymer surface **210.** In a preferred embodiment of this method when using a thermoplastic polyimide adhesive tape, the polyimide surface is not enough hydrophilic, which causes that the dispensed liquid acquires a semispherical shape even after several trials of spreading. This behavior is not convenient for subsequent steps of the method. Consequently, a previous functionalization of the semi-transparent polyimide surface is needed for making this surface more hydrophilic. The polyimide functionalization is accomplished introducing the donor substrate into a vacuum sample desiccator to extract as much as possible the oxygen content by means of a mechanical pump. Subsequently, the desiccator containing the donor substrate is submitted to a microwave-oven-generated plasma using 1000 W during 5 s. After this process the polyimide surface is ready to receive the liquid specimen.

Different liquid dispensers can be used to dispense the liquid specimen onto the donor substrate. Some examples of dispensers are pipettes, tips, needles and syringes. In a preferred embodiment of this method, a micropipette is employed to dispense between 5 to 15 µL of the liquid specimen **400** containing the enriched population of CTCs or DTCs. The spreading of the liquid specimen can be carried out using specific tools to spread liquids onto flat surfaces such as blades and rolls. Nevertheless, some inconveniences related to recovering the remaining liquid in the tool are unavoidable. To minimize this difficulty, the liquid specimen can be spread with the tip of the same micropipette used to dispense the liquid specimen. In another preferred embodiment of this method, vibrations can be generated in the dispensed liquid by means of for example ultrasounds to provoke the spreading of the liquid specimen until to cover an area sufficiently extended. In a preferred embodiment of this method an area ranging between 10 to 200 mm² is covered forming a liquid film with a thickness ranging between 30 to 70 µm. Figure 4 shows a schematic illustration of the donor substrate with liquid specimen **400** spread on the semi-transparent polymeric adhesive tape **200.**

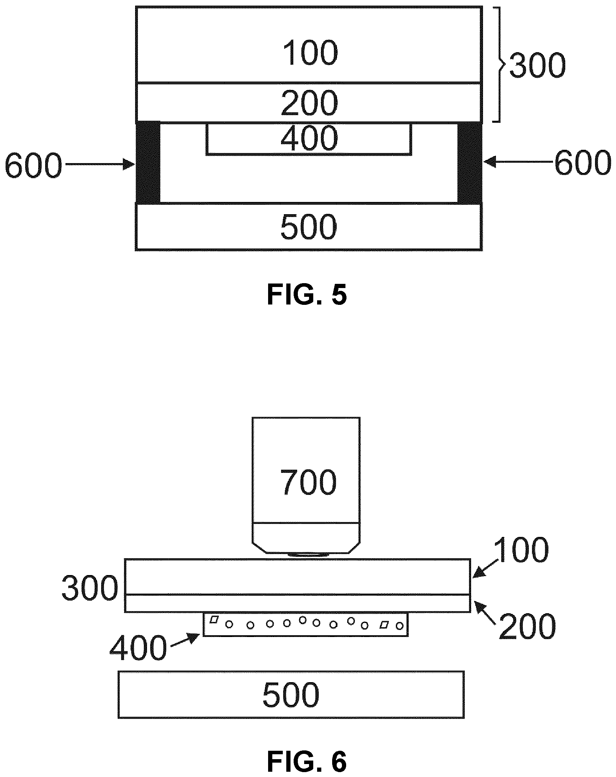

A wide variety of reservoirs, plates, dishes, slides, sheets, liquid reservoirs such multi-well plates and tubes can be employed as receiving substrates in this method. The only restriction involving the receiving substrate arrangement is related to the separation distance needed between this substrate and the liquid specimen. Such separation distance can range between 0.1 and 20 mm. In accordance with following developments of the method, the receiving substrate is located in front of the donor substrate, in such a way that the part of the liquid propelled, as a consequence of the laser impact, can be deposited onto the receiving substrate. In a preferred embodiment of the method, the donor substrate **300** is arranged with the liquid specimen facing down respect to the receiving substrate as shown in Figure 5. The separation distance between the liquid specimen and the receiving substrate can be accomplished by holding both substrates separately and controlling the separation between them by means of stage controllers. Other strategy consists on employing the same holding for both receiving and donor substrates and inserting two pieces of a flat material, necessarily thicker than the liquid specimen, between both substrates. These pieces are placed on the extremes of both substrates avoiding contact with the liquid specimen. In a preferred embodiment of this method, two pieces of crystalline silicon of about 550 µm of thickness are used as separators between the substrates donor and receiving. In another preferred embodiment of this method, two pieces of polymeric adhesive tape are used as separator. Therefore, in this method, the separation distance can ranged depending on the thickness of the material used as separator (between 0.1 and 2 mm preferably). Figure **5** illustrates the location of the two separation pieces **600** interposed between the donor **300** and receiving **500** substrates.

A microscopy system allowing the simultaneous inspection of the liquid specimen in the optical and fluorescence fields is employed. In a preferred embodiment of this method, the same objective lens **700** with a sub-micrometric precision is used for the optical and fluorescence inspection, in such a way that two simultaneous images of the same field view, one optical and the other fluorescent, can be obtained from the liquid specimen. Figure 6 shows the arrangement of the liquid specimen with respect to the objective lens of the optical and fluorescence microscopy system. A linear displacement system comprising two separate translation controllers is used to independently move the donor substrate **300** containing the liquid specimen spread on it and the receiving substrate **500** with respect to the objective lens **700.** In a preferred embodiment of this method, two motorized linear stages with micrometric precision are used to independently translate the donor substrate **300** containing the liquid specimen **400** and the receiving substrate **500** so that the liquid specimen can be inspected through a fixed objective lens.

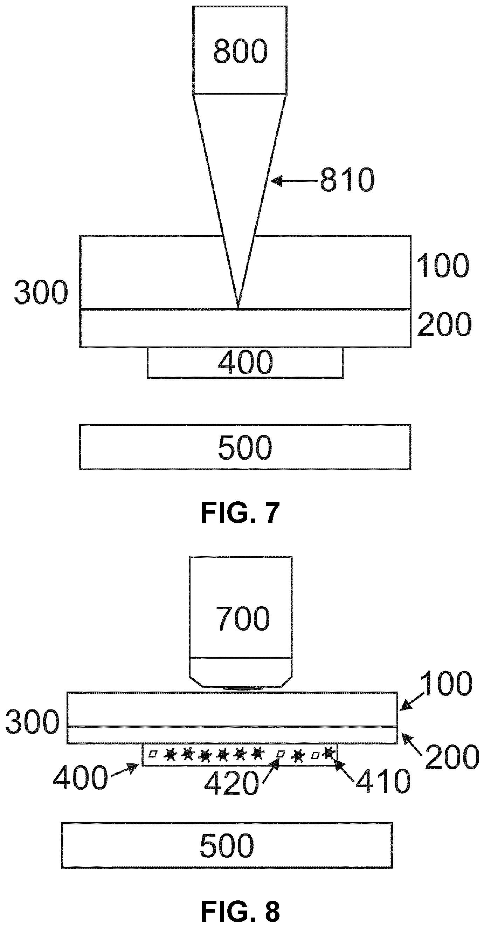

A laser beam **810** is focalized on the interface between the transparent support **100** and the semi-transparent thermoplastic polymeric adhesive tape **200.** The laser radiation is absorbed by the semi-transparent thermoplastic polymeric adhesive tape generating a vapor pocket whose pressure produces the debonding of the polymeric adhesive tape from the transparent support and the deformation in the thermoplastic polymer, called blister, which can mechanically propels a droplet of liquid, depending on the laser energy, onto the receiving substrate. Each propelled droplet contains a single cell or a cluster of cells, so the laser-assisted transfer process induces the isolation of the selected cell from the liquid specimen. Figure **7** illustrates the arrangement of the laser source **800** and the focalized laser beam **810** with respect to the donor **300** and receiving substrates **500.** In a preferred embodiment of this method, the laser source is an ultraviolet multi-frequency pulsed laser operating in the nanosecond regime. A 355 nm laser focalized to obtain a range of energy density between 0.3 and 50 J/cm² is used in a preferred embodiment of this method. The tuning of the laser energy density gives place to different work regimes in terms of the effects caused by the mechanical impulse generated in the blister formation on the selected cell or cell clusters. In first place, a threshold value of energy density leading to droplet transfer and consequently to cell transfer is determined. Values of energy density very above this threshold are considered inefficient, since they would induce cellular damage and contamination due cell direct exposition to the laser irradiation and possible polymer releasing. Values close to the threshold are considered the optimal work regimen for the cell isolation. Finally, values close to but below the threshold can be used to generate certain perturbation in the liquid not involving drop transfer. In this case, the generated blister lead to a movement of cells within the liquid specimen. If a cluster of cells is perturbed under these specific energy conditions, the generated blister can induce a disaggregation of the selected clusters of cells, which is convenient for a subsequent single cell isolation.

It is important to mention that in the optimal regimen, the laser energy can be tuned in order to transfer higher volume of liquid containing cell aggregations or clusters so not only single cell but also cell clusters can be transferred. In a preferred embodiment of this method, when working with a 355 nm 10 ns pulsed laser and a 25 µm semitransparent polymeric adhesive tape, the optimal energy density range is found between 1.0 to 1.8 J/cm². Energy densities between 0.3 and 1.0 J/cm² would induce a separation of clusters into different single cells without propelling any cell to the receiving substrate. Finally, energy densities higher than 1.8 J/cm² could induce thermal damage of liquid specimen.

In this method, there are two options of laser-assisted transfer according to the cell populations that can be tumor cells and non-tumor cells. In the first option the tumor cells are transferred whereas in the second option the non-tumor cells are transferred. In either option, two further alternatives exist depending on the fluorescence condition of the cells. If the cells are fluorescently stained their identification is straightforward from a fluorescence microscopy inspection. On the other hand, when transferring non-stained cells, a comparison between simultaneous optical and fluorescence images of the same field view is required in order to identify the location of the cell to be transferred.

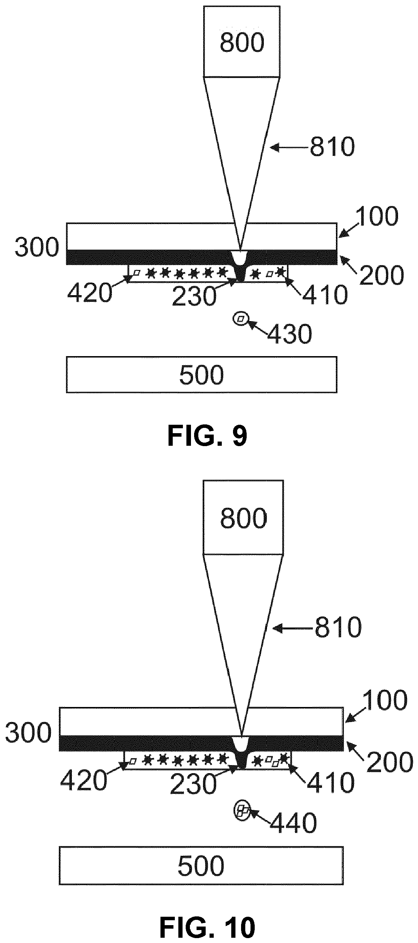

Figure **8** shows among other elements a schematic illustration of the two populations of cells when one of them is fluorescently stained. In this case, non-tumor cells are represented as fluorescently stained **410** while tumor cells are represented as non-stained **420.** In order to isolate the most interesting population which is the tumor cells (CTCs and DTCs) the previous mentioned optical and fluorescence comparison is needed to identify the locations of such cells. In this particular case, the tumor cells have kept unaltered after the enrichment and staining processes. The laser-assisted transfer isolation of a tumor cell in this particular condition is equivalent to the well-known negative selection process of CTCs and DTCs and therefore, it is called **laser negative selection** in this disclosure. Figure **9** shows among other elements a schematic illustration of the liquid specimen **400** where the non-tumor cells are represented as fluorescently stained cells **410** while the tumor cells **420** are represented as non-stained. Thus, the laser impinging on a specific location of a non-stained tumor cell **420** gives place to a blister **230** in the semi-transparent polymeric adhesive tape that propels a droplet of the liquid specimen **430** containing a single tumor cell in such a way that, the laser negative selection is accomplished. In a preferred embodiment of this method, a single pulse of an UV laser system working in the nanosecond regime leads to the ejection of a single droplet with a volume ranging from 1 to 500 pL and containing a single non-stained CTC or DTC. When the tumor cells are fluorescently stained, the process of selection and isolation is equivalent to the well-known positive selection process for CTCs and DTCs. Thus, when the laser-assisted transfer is applied to isolate fluorescently stained tumor cells, the term laser positive selection is adopted to describe this process. In addition, this method also allows transferring the non-tumor cells, leaving the tumor cells in the donor substrate. Due to the fact that, a simple energy tuning enables the method to transfer higher volumes of liquid, the previous description is also applicable to clusters of cells. Figure **10** shows a schematic illustration of the laser negative selection of a cluster of non-stained tumor cells **440.** In this case, laser energy must be adjusted to allow transferring enough amount of liquid comprising the aggregation of two or more tumor cells.

## Claims

1. A method for detecting and manipulating tumor cells found in organic fluids comprising the steps of:
a) providing a liquid specimen (400) of the organic fluid containing an enriched population of tumor cells and a population of non-tumor cells;
b) fluorescently staining at least one of the two cell populations wherein a different fluorescence dye is used for each population when both populations are stained;
c) providing a donor substrate (300) comprising a transparent support (100) and a semi-transparent polymeric adhesive tape (200) adhered to the transparent support (100);
d) dispensing and spreading the liquid specimen (400) onto the semi-transparent polymeric adhesive tape (200);
e) providing a receiving substrate (500);
f) placing the receiving substrate (500) facing the surface of the donor substrate (300) where the liquid specimen (400) is spread maintaining a separation distance between them;
g) visualizing the same portion of the liquid specimen (400) by means of optical and fluorescence microscopy (700);
h) comparing the optically and fluorescently obtained images to identify the location of tumor cells or non-tumor cells;
i) providing a laser beam (810) focused on the interface between the transparent support (100) and the semi-transparent polymeric adhesive tape (200) of the donor substrate (300);
j) directing the laser beam (810) to the identified location of at least one tumor cell or at least one non-tumor cell; so that the laser beam (810) energy is absorbed by the semi-transparent polymeric adhesive tape (200) at the identified location; giving place to the formation of a blister on the polymeric adhesive tape (200) that generates a mechanical perturbation in the liquid specimen (400) leading to the movement of at least one cell or cluster of cells.

2. The method according to claim 1, wherein the blister mechanically propels a portion of the liquid specimen (400) towards the receiving substrate (500), the propelled liquid comprising at least one tumor cell without any non-tumor cells.

3. The method according to claim 1, wherein the blister mechanically propels a portion of the liquid specimen (400) towards the receiving substrate (500), the propelled liquid comprising at least one non-tumor cell without any tumor cells.

4. The method according to claims 2 or 3, wherein the portion of the liquid specimen (400) propelled by the laser beam (810) comprises a single cell.

5. The method according to claims 2 or 3, wherein the portion of the liquid specimen (400) propelled by the laser beam (810) comprises a cluster of cells.

6. The method according to any of the claims 2 to 5, wherein only the tumor cells or only the non-tumor cells are stained.

7. The method according to claim 6, wherein the portion of the liquid specimen (400) propelled by the laser beam (810) comprises at least one non-stained cell.

8. The method according to claim 6, wherein the portion of the liquid specimen (400) propelled by the laser beam (810) comprises at least one stained cell.

9. The method according to claim 1, wherein the generated blister leads to the disaggregation of a cluster of cells.

10. The method according to any preceding claim, wherein tumor cells comprise at least one of circulating tumor cells and disseminated tumor cells.

11. The method according to any preceding claim, wherein the non-tumor cells comprise at least one of hematopoietic cells and white blood cells.

12. A system for detecting, manipulating and isolating tumor cells from an organic fluid comprising:
a donor substrate (300) comprising a transparent support (100) and a semi-transparent polymeric adhesive tape (200) adapted to be adhered on one side of the transparent support (100) and further adapted to receive, on the side opposite to the transparent support (100), a liquid specimen (400) of the organic fluid containing an enriched population of tumor cells and a population of non-tumor cells wherein at least one of the two cell populations are fluorescently stained,
a receiving substrate (500) arranged at a distance from the donor substrate (300) while facing the semi-transparent polymeric adhesive tape (200);
a visualization system adapted to provide a visualization of the same portion of the liquid specimen (400) by means of optical and fluorescence microscopy and further adapted to compare the optically and fluorescently obtained images,
a laser system adapted to focus a laser beam (810) on the interface between the transparent support (100) and the semi-transparent polymeric adhesive tape (200) of the donor substrate (300) and further adapted to direct the laser beam (810) to the location of at least one tumor cell or at least one non-tumor cell so that the laser beam (810) energy is absorbed by the semi-transparent polymeric adhesive tape (200) giving place to the formation of a blister on the semi-transparent polymeric adhesive tape (200) that generates a mechanical perturbation in the liquid specimen (400) leading to the movement of at least one cell or cluster of cells.

13. A system for detecting, manipulating and isolating tumor cells from an organic fluid according to claim 12, wherein the semi-transparent polymeric adhesive tape (200) comprises at least one thermoplastic polyimide sheet.

14. A system for detecting, manipulating and isolating tumor cells from an organic fluid according to claims 12 or 13 wherein the semi-transparent polymeric adhesive tape (200) comprises at least one layer of a transparent adhesive silicone.

## Patentansprüche

1. Ein Verfahren zum Detektieren und Manipulieren von Tumorzellen, die in organischen Flüssigkeiten gefunden werden, umfassend die Schritte:
a) Bereitstellen einer flüssigen Probe (400) der organischen Flüssigkeit, die eine angereicherte Population von Tumorzellen und eine Population von Nicht-Tumorzellen enthält;
b) Fluoreszenzfärben mindestens einer der beiden Zellpopulationen, wobei ein unterschiedlicher Fluoreszenzfarbstoff für jede Population verwendet wird, wenn beide Populationen gefärbt sind;
c) Bereitstellen eines Donorsubstrats (300), umfassend einen transparenten Träger (100) und ein semitransparentes polymeres Klebeband (200), das an dem transparenten Träger (100) haftet;
d) Ausgeben und Verteilen der flüssigen Probe (400) auf das semitransparente polymere Klebeband (200);
e) Bereitstellen eines Aufnahmesubstrats (500);
f) Anordnen des Aufnahmesubstrats (500), das der Oberfläche des Donorsubstrats (300) zugewandt ist, wo die flüssige Probe (400) verteilt ist, unter Beibehaltung eines Abstands zwischen ihnen;
g) Visualisieren des gleichen Abschnitts der flüssigen Probe (400) mittels optischer und Fluoreszenzmikroskopie (700);
h) Vergleichen der optisch und fluoreszierend erhaltenen Bilder, um die Position von Tumorzellen oder Nicht-Tumorzellen zu identifizieren;
i) Bereitstellen eines Laserstrahls (810), der auf die Grenzfläche zwischen dem transparenten Träger (100) und dem semitransparenten Polymerklebeband (200) des Donorsubstrats (300) fokussiert ist;
j) Richten des Laserstrahls (810) auf die identifizierte Position mindestens einer Tumorzelle oder mindestens einer Nicht-Tumorzelle; so dass die Energie des Laserstrahls (810) durch das semitransparente polymere Klebeband (200) an der identifizierten Position absorbiert wird; Platzieren der Bildung eines Blisters auf dem polymeren Klebeband (200), das eine mechanische Störung in der flüssigen Probe (400) erzeugt, die zu der Bewegung mindestens einer Zelle oder eines Clusters von Zellen führt.

2. Das Verfahren nach Anspruch 1, wobei der Blister einen Teil der flüssigen Probe (400) mechanisch in Richtung des Aufnahmesubstrats (500) treibt, wobei die getriebene Flüssigkeit mindestens eine Tumorzelle ohne Nicht-Tumorzellen umfasst.

3. Das Verfahren nach Anspruch 1, wobei der Blister einen Teil der flüssigen Probe (400) mechanisch in Richtung des Aufnahmesubstrats (500) treibt, wobei die getriebene Flüssigkeit mindestens eine Nicht-Tumorzelle ohne Tumorzellen umfasst.

4. Das Verfahren nach Anspruch 2 oder 3, wobei der Teil der flüssigen Probe (400), der durch den Laserstrahl (810) angetrieben wird, eine einzelne Zelle umfasst.

5. Das Verfahren nach Anspruch 2 oder 3, wobei der Teil der flüssigen Probe (400), der durch den Laserstrahl (810) angetrieben wird, eine Gruppe von Zellen umfasst.

6. Das Verfahren nach einem der Ansprüche 2 bis 5, wobei nur die Tumorzellen oder nur die Nicht-Tumorzellen gefärbt werden.

7. Das Verfahren nach Anspruch 6, wobei der Teil der flüssigen Probe (400), der durch den Laserstrahl (810) angetrieben wird, mindestens eine nicht verschmutzte Zelle umfasst.

8. Das Verfahren nach Anspruch 6, wobei der Teil der flüssigen Probe (400), der durch den Laserstrahl (810) angetrieben wird, mindestens eine gefärbte Zelle umfasst.

9. Das Verfahren nach Anspruch 1, wobei der erzeugte Blister zur Disaggregation eines Zellclusters führt.

10. Das Verfahren nach einem der vorhergehenden Ansprüche, wobei Tumorzellen mindestens eine der zirkulierenden Tumorzellen und disseminierten Tumorzellen umfassen.

11. Das Verfahren nach einem der vorhergehenden Ansprüche, wobei die Nicht-Tumorzellen mindestens eine von hämatopoetischen Zellen und weißen Blutzellen umfassen.

12. Ein System zum Detektieren, Manipulieren und Isolieren von Tumorzellen aus einer organischen Flüssigkeit, umfassend:
ein Donorsubstrat (300), das einen transparenten Träger (100) und ein semitransparentes polymeres Klebeband (200) umfasst, das dazu ausgelegt ist, auf einer Seite des transparenten Trägers (100) geklebt zu werden, und ferner dazu ausgelegt ist, auf der Seite gegenüber dem transparenten Träger (100) eine flüssige Probe (400) der organischen Flüssigkeit aufzunehmen, die eine angereicherte Population von Tumorzellen und eine Population von Nicht-Tumorzellen enthält, wobei mindestens eine der beiden Zellpopulationen fluoreszierend gefärbt ist,
ein Aufnahmesubstrat (500), das in einem Abstand von dem Donorsubstrat (300) angeordnet ist, während es dem semitransparenten Polymerklebeband (200) zugewandt ist;
ein Visualisierungssystem, das angepasst ist, um eine Visualisierung desselben Abschnitts der flüssigen Probe (400) mittels optischer und Fluoreszenzmikroskopie bereitzustellen, und das ferner angepasst ist, um die optisch und fluoreszierend erhaltenen Bilder zu vergleichen,
ein Lasersystem, das angepasst ist, um einen Laserstrahl (810) auf die Grenzfläche zwischen dem transparenten Träger (100) und dem semitransparenten polymeren Klebeband (200) des Donorsubstrats (300) zu fokussieren, und das ferner angepasst ist, um den Laserstrahl (810) zu der Position von mindestens einer Tumorzelle oder mindestens einer Nicht-Tumorzelle zu lenken, so dass die Energie des Laserstrahls (810) durch das semitransparente polymere Klebeband (200) absorbiert wird, was Platz für die Bildung eines Blisters auf dem semitransparenten polymeren Klebeband (200) gibt, das eine mechanische Störung in der flüssigen Probe (400) erzeugt, die zu der Bewegung von mindestens einer Zelle oder einer Gruppe von Zellen führt.

13. Ein System zum Detektieren, Manipulieren und Isolieren von Tumorzellen aus einer organischen Flüssigkeit nach Anspruch 12, wobei das semitransparente polymere Klebeband (200) mindestens eine thermoplastische Polyimidfolie umfasst.

14. Ein System zum Detektieren, Manipulieren und Isolieren von Tumorzellen aus einer organischen Flüssigkeit nach Anspruch 12 oder 13, wobei das semitransparente polymere Klebeband (200) mindestens eine Schicht aus einem transparenten klebenden Silikon umfasst.

## Revendications

1. Procédé pour détecter et manipuler des cellules tumorales trouvées dans des fluides organiques, comprenant les étapes consistant à :
a) fournir un échantillon liquide (400) du fluide organique contenant une population enrichie de cellules tumorales et une population de cellules non tumorales ;
b) la coloration fluorescente d'au moins une des deux populations cellulaires, un colorant fluorescent différent étant utilisé pour chaque population lorsque les deux populations sont colorées ;
c) fourni un substrat donneur (300) comprenant un support transparent (100) et un ruban adhésif polymère semi-transparent (200) collé au support transparent (100) ;
d) la distribution et l'épandage de l'échantillon liquide (400) sur le ruban adhésif polymère semi-transparent (200) ;
e) fournir un substrat de réception (500) ;
f) le placement du substrat récepteur (500) face à la surface du substrat donneur (300) où l'échantillon liquide (400) est étalé en maintenant une distance de séparation entre eux ;
g) la visualisation de la même partie de l'échantillon liquide (400) au moyen d'une microscopie optique et à fluorescence (700) ;
h) comparer les images obtenues optiquement et par fluorescence pour identifier l'emplacement de cellules tumorales ou de cellules non tumorales ;
i) fournir un faisceau laser (810) focalisé sur l'interface entre le support transparent (100) et la bande adhésive polymère semi-transparente (200) du substrat donneur (300) ;
j) dirige le faisceau laser (810) vers l'emplacement identifié d'au moins une cellule tumorale ou d'au moins une cellule non tumorale; de sorte que l'énergie du faisceau laser (810) soit absorbée par la bande adhésive polymère semi-transparente (200) à l'emplacement identifié; laisser la place à la formation d'une cloque sur la bande adhésive polymère (200) qui génère une perturbation mécanique dans l'échantillon liquide (400) conduisant au mouvement d'au moins une cellule ou d'un groupe de cellules.

2. Procédé selon la revendication 1, dans lequel le blister propulse mécaniquement une partie de l'échantillon liquide (400) vers le substrat récepteur (500), le liquide propulsé comprenant au moins une cellule tumorale sans aucune cellule non tumorale.

3. Procédé selon la revendication 1, dans lequel le blister propulse mécaniquement une partie de l'échantillon liquide (400) vers le substrat récepteur (500), le liquide propulsé comprenant au moins une cellule non tumorale sans aucune cellule tumorale.

4. Procédé selon la revendication 2 ou 3, dans lequel la partie de l'échantillon liquide (400) propulsée par le faisceau laser (810) comprend une seule cellule.

5. Procédé selon la revendication 2 ou 3, dans lequel la partie de l'échantillon liquide (400) propulsée par le faisceau laser (810) comprend un groupe de cellules.

6. Procédé selon l'une quelconque des revendications 2 à 5, dans lequel seules les cellules tumorales ou seules les cellules non tumorales sont colorées.

7. Procédé selon la revendication 6, dans lequel la partie de l'échantillon liquide (400) propulsée par le faisceau laser (810) comprend au moins une cellule non colorée.

8. Procédé selon la revendication 6, dans lequel la partie de l'échantillon liquide (400) propulsée par le faisceau laser (810) comprend au moins une cellule colorée.

9. Procédé selon la revendication 1, dans lequel la cloque générée conduit à la désagrégation d'un groupe de cellules.

10. Procédé selon l'une quelconque des revendications précédentes, dans lequel les cellules tumorales comprennent au moins l'une des cellules tumorales circulantes et des cellules tumorales disséminées.

11. Procédé selon l'une quelconque des revendications précédentes, dans lequel les cellules non tumorales comprennent au moins l'une des cellules hématopoïétiques et des globules blancs.

12. Système pour détecter, manipuler et isoler des cellules tumorales à partir d'un fluide organique comprenant :
un substrat donneur (300) comprenant un support transparent (100) et un ruban adhésif polymère semi-transparent (200) adapté pour être collé sur un côté du support transparent (100) et adapté en outre pour recevoir, sur le côté à l'opposé du support transparent (100), un échantillon liquide (400) du fluide organique contenant une population enrichie de cellules tumorales et une population de cellules non tumorales dans lesquelles au moins une des deux populations cellulaires est colorée par fluorescence,
un substrat de réception (500) disposé à une certaine distance du substrat donneur (300) tout en faisant face à la bande adhésive polymère semi-transparente (200) ;
un système de visualisation adapté pour fournir une visualisation de la même partie de l'échantillon liquide (400) au moyen d'une microscopie optique et par fluorescence et adapté en outre pour comparer les images obtenues optiquement et par fluorescence,
un système laser adapté pour focaliser un faisceau laser (810) sur l'interface entre le support transparent (100) et la bande adhésive polymère semi-transparente (200) du substrat donneur (300) et adapté en outre pour diriger le faisceau laser (810) vers l'emplacement d'au moins une cellule tumorale ou d'au moins une cellule non tumorale de sorte que l'énergie du faisceau laser (810) soit absorbée par la bande adhésive polymère semi-transparente (200) donnant lieu à la formation d'une cloque sur la bande adhésive polymère semi-transparente (200) qui génère une perturbation mécanique dans l'échantillon liquide (400) conduisant au mouvement d'au moins une cellule ou d'un groupe de cellules.

13. Système pour détecter, manipuler et isoler des cellules tumorales à partir d'un fluide organique selon la revendication 12, dans lequel le ruban adhésif polymère semi-transparent (200) comprend au moins une feuille de polyimide thermoplastique.

14. Système pour détecter, manipuler et isoler des cellules tumorales à partir d'un fluide organique selon la revendication 12 ou 13, dans lequel le ruban adhésif polymère semi-transparent (200) comprend au moins une couche d'une silicone adhésive transparente.
